(19) Europäisches Patentamt
European Patent Office
Office européen des brevets

(11) Publication number: 0 220 878 A2

# (12) EUROPEAN PATENT APPLICATION

(21) Application number: 86307975.2

(22) Date of filing: 15.10.86

(51) Int. Cl.[4]: C 07 C 93/08
C 07 C 93/10, C 07 C 93/14,
A 61 K 31/135

(30) Priority: 16.10.85 GB 8525483

(43) Date of publication of application:
06.05.87 Bulletin 87/19

(84) Designated Contracting States:
AT BE CH DE ES FR GB GR IT LI LU NL SE

(71) Applicant: GLAXO GROUP LIMITED
Clarges House 6-12 Clarges Street
London W1Y 8DH (GB)

(72) Inventor: Finch, Harry
19 Hensley Close
Hitchin Hertfordshire (GB)

Naylor, Alan
35 Layston Park
Royston Hertfordshire (GB)

Lunts, Lawrence Henry Charles
10 Wromley West End
Broxbourne Hertfordshire (GB)

Skidmore, Ian Frederick
2 Wendover Drive
Welwyn Hertfordshire (GB)

(74) Representative: Marchant, James Ian et al
Elkington and Fife High Holborn House 52/54 High Holborn
London WC1V 6SH (GB)

## (54) Ethanolamine compounds.

(57) The invention provides compounds of the general formula (I)

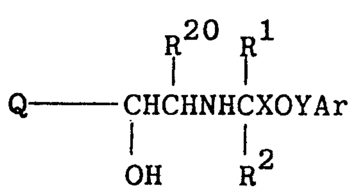

(I)

wherein
Ar represents a phenyl group optionally substituted by one or more substituents selected from halogen atoms, or the groups $C_{1-6}$ alkyl, nitro, $-(CH_2)_qR$, [where R is hydroxy, $C_{1-6}$ alkoxy, $-NR^3R^4$ (where $R^3$ and $R^4$ each represents a hydrogen atom, or a $C_{1-4}$ alkyl group, or $-NR^3R^4$ forms a saturated heterocyclic amino group which has 5-7 ring members and optionally contains in the ring one or more atoms selected from -O- or -S- or a group -NH- or $-N(CH_3)-$), $-NR^5COR^6$ (where $R^6$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$alkoxy or $-NR^3R^4$), $-SR^9$ (where $R^9$ is a hydrogen atom, or a $C_{1-4}$ alkyl or phenyl group), $-SOR^9$, $-SO_2R^9$, or -CN, and q represents an integer from 0 to 3], or $-O(CH_{2t}R^{11}$ [where $R^{11}$ represents a hydroxy or $C_{1-4}$ alkoxy group and t is 2 or 3], or Ar is a phenyl group substituted by an alkylenedioxy group of formula $-O(CH_2)_pO-$, where p represents 1 or 2;
$R^1$ and $R^2$ each represent a hydrogen atom or a $C_{1-3}$ alkyl group, with the proviso that the sum total of carbon atoms in $R^1$ and $R^2$ is not more than 4;
$R^{20}$ represents a $C_{1-3}$ alkyl group;
X represents a $C_{2-8}$ alkylene $C_{2-8}$ alkenylene or $C_{2-8}$alkynylene chain, and
Y represents a $C_{1-7}$ alkylene $C_{2-7}$ alkenylene or $C_{2-7}$alkynylene chain, with the proviso that the sum total of carbon atoms in X and Y is 4 to 12;
Q represents (a)

EP 0 220 878 A2

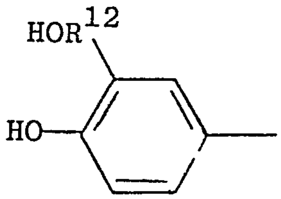

EMI PA=2 FR=1 HE=30 WI=40 TI=CHE

(where $R^{12}$ is a straight or branched $C_{1-3}$ alkylene group),

(b) 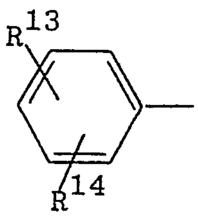

(where one of $R^{13}$ and $R^{14}$ is a hydroxy group and the other is a hydrogen or halogen atom or a hydroxy group),

(c) 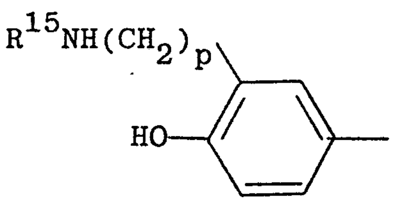

[where $R^{15}$ is a group $R^{16}CO$-, $R^{16}NHCO$-, $R^{16}R^{17}NSO_2$- or $R^{18}SO_2$- (where $R^{16}$ and $R^{17}$ is each a hydrogen atom or a $C_{1-3}$alkyl group and $R^{18}$ is a $C_{1-3}$ alkyl group) and p is an integer 0 or 1],

(d) 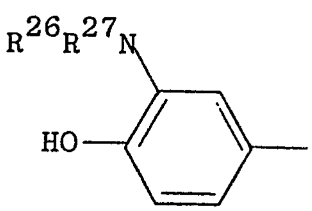

(where $R^{26}$ and $R^{27}$ is each a hydrogen atom or a $C_{1-3}$ alkyl group or, when $R^{26}$ is a hydrogen atom, $R^{27}$ may also be a $C_{1-4}$alkoxycarbonyl group),

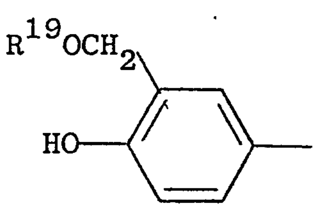

(where $R^{19}$ is a $C_{1-3}$ alkyl group),

(f) 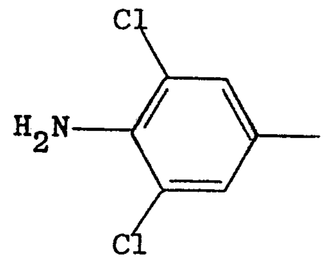

(g) 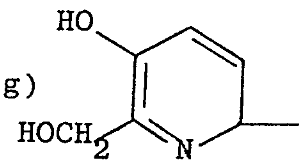

(h) 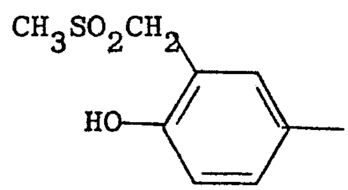 or (i)

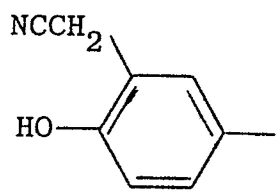 ;

and physiologically acceptable salts and solvates (e.g. hydrates) thereof.

The compounds have a stimulant action at $\beta_2$-adrenoreceptors and may be used in the treatment of diseases associated with reversible airways obstruction such as asthma and chronic bronchitis.

**Description**

Ethanolamine Compounds

This invention relates to ethanolamine compounds having a stimulant action at $\beta_2$-adrenoreceptors, to processes for their preparation, to pharmaceutical compositions containing them and to their use in medicine.

Ethanolamine derivatives of the general structure

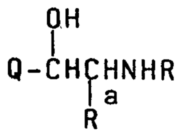

in which Q represents groupings of the type described hereinafter, $R_a$ represents inter alia an alkyl group and R represents inter alia an alkyl, aralkyl or aryloxyalkyl group have previously been described as bronchodilators having stimulant activity at β-adrenreceptors. We have now found a novel group of ethanolamine derivatives which differ in structure from those described previously, and have a desirable and potentially useful profile of activity.

Thus the present invention provides compounds of the general formula (I)

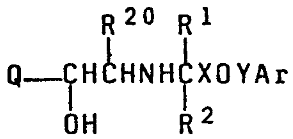

(I)

wherein

Ar represents a phenyl group optionally substituted by one or more substituents selected from halogen atoms, or the groups $C_{1-6}$ alkyl, nitro, $-(CH_2)_qR$, [where R is hydroxy, $C_{1-6}$ alkoxy, $-NR^3R^4$ (where $R^3$ and $R^4$ each represents a hydrogen atom, or a $C_{1-4}$ alkyl group, or $-NR\ ^3R^4$ forms a saturated heterocyclic amino group which has 5-7 ring members and optionally contains in the ring one or more atoms selected from -O- or -S- or a group -NH- or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$alkoxy or $-NR^3R^4$), $-SR^9$ (where $R^9$ is a hydrogen atom, or a $C_{1-4}$ alkyl or phenyl group), $-SOR^9$, $-SO_2R^9$, or -CN, and q represents an integer from 0 to 3], or $-O(CH_2)_tR^{11}$ [where $R^{11}$ represents a hydroxy or $C_{1-4}$ alkoxy group and t is 2 or 3], or Ar is a phenyl group substituted by an alkylenedioxy group of formula $-O(CH_2)_pO-$, where p represents 1 or 2;

$R^1$ and $R^2$ each represent a hydrogen atom or a $C_{1-3}$ alkyl group, with the proviso that the sum total of carbon atoms in $R^1$ and $R^2$ is not more than 4;

$R^{20}$ represents a $C_{1-3}$ alkyl group;

X represents a $C_{2-8}$ alkylene $C_{2-8}$ alkenylene or $C_{2-8}$alkynylene chain, and

Y represents a $C_{1-7}$ alkylene $C_{2-7}$ alkenylene or $C_{2-7}$alkynylene chain, with the proviso that the sum total of carbon atoms in X and Y is 4 to 12;

Q represents (a)

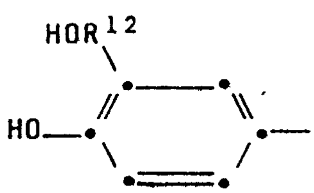

(where $R^{12}$ is a straight or branched $C_{1-3}$ alkylene group),

(b) 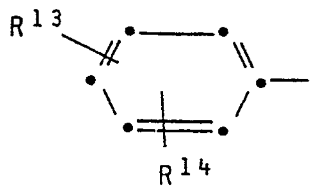

(where one or $R^{13}$ and $R^{14}$ is a hydroxy group and the other is a hydrogen or halogen atom or a hydroxy group),

(c) 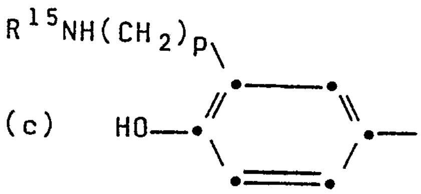

[where $R^{15}$ is a group $R^{16}CO$-, $R^{16}NHCO$-, $R^{16}R^{17}NSO_2$- or $R^{18}SO_2$- (where $R^{16}$ and $R^{17}$ is each a hydrogen atom or a $C_{1-3}$ alkyl group and $R^{18}$ is a $C_{1-3}$ alkyl group) and p is an integer 0 or 1],

(d) 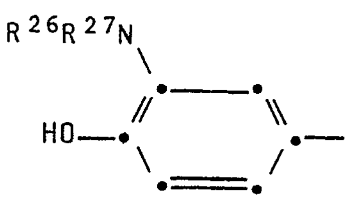

(where $R^{26}$ and $R^{27}$ is each a hydrogen atom or a $C_{1-3}$ alkyl group or, when $R^{26}$ is a hydrogen atom, $R^{27}$ may also be a $C_{1-4}$ alkoxycarbonyl group),

(e) 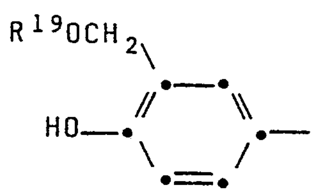

(where $R^{19}$ is a $C_{1-3}$ alkyl group),

(f) 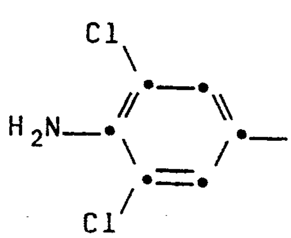

(g) 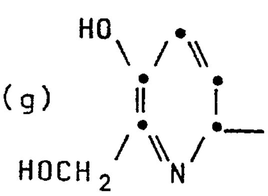

(h) 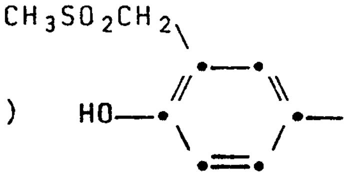 or (i) 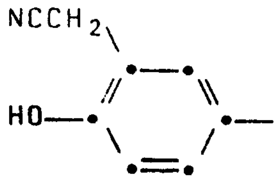 ;

and physiologically acceptable salts and solvates (e.g. hydrates) thereof.

It will be appreciated that the compounds of general formula (I) possess one or more asymmetric carbon atoms.

The compounds according to the invention thus include all enantiomers, diastereoisomers and mixtures thereof, including racemates. Compounds in which the carbon atom in the $-\underset{OH}{CH}-$ group is in the R configuration are preferred.

When $-NR^3R^4$ in compounds of formula (I) represents a saturated heterocyclic amino group this may be for example a pyrrolidino, piperidino, hexamethylenimino, piperazino, N-methylpiperazino, morpholino, homomorpholino or thiamorpholino group.

In the compounds of formula (I) Ar may be for example a phenyl group. Examples of the optional substituents which may be present on the phenyl group represented by Ar include chlorine, bromine, iodine, fluorine, methyl, ethyl, ethoxy, $-(CH_2)_qR$ [where R is hydroxy, methoxy, amino, methylamino, ethylamino, dimethylamino, diethylamino, morpholino, piperidino, piperazino, N-methylpiperazino, $-NHCOR^6$ (where $R^6$ is hydrogen or $C_{1-4}$alkyl (e.g. methyl, ethyl, isopropyl or n-butyl), $C_{1-4}$alkoxy (e.g. methoxy, ethoxy, isopropoxy or n-butoxy), phenyl, amino or N,N-dimethylamino), $-N(CH_3)COCH_3$, $-NR^5SO_2R^7$, (where $R^5$ represents a hydrogen atom or a methyl group and $R^7$ represents methyl, butyl, phenyl, amino or dimethylamino), -COOH, $-COOCH_3$, $-COOH_2CH_2CH_3$, $-CONH_2$, $-CON(CH_3)_2$, $-CON(CH_2CH_3)_2$, $-CON(CH_2CH_2CH_3)_2$, $-SR^9$ (where $R^9$ is methyl, ethyl or phenyl) $-SOCH_3$, $-SO_2CH_3$, or CN, and q is zero, 1, 2 or 3], $-NO_2$, $-O(CH_2)_2OH$, $-O(CH_2)_3OH$, $-O(CH_2)_2OCH_3$, or $-O(CH_2)_2OCH_2CH_3$.

The phenyl groups represented by Ar may for example contain one, two or three substituents, which may be present at the 2-, 3-, 4-, 5- or 6-positions on the phenyl ring.

The group Ar preferably represents a phenyl group optionally carrying one substituent.

In the compounds of formula (I) $R^1$, $R^2$ and $R^{20}$ may each be for example methyl, ethyl, propyl or isopropyl groups except that if one is an ethyl, propyl or isopropyl group then at least one of $R^1$ and $R^2$ is a hydrogen atom. Thus for example $R^1$ may be a hydrogen atom or a methyl group and $R^2$ may be a hydrogen atom. The group $R^{29}$ preferably represents a methyl or ethyl group.

The group X in formula (I) may for example contain 3 to 8 carbon atoms and the chain Y may contain for example from 2 to 7 carbon atoms.

X may be for example $-(CH_2)_2-$, $-(CH_2)_3-$, $-(CH_2)_4-$, $-(CH_2)_5-$, $-(CH_2)_6-$, $-CH_2C\equiv CCH_2-$, $-(CH_2)_2CH=CHCH_2-$, $-(CH_2)_2C\equiv CCH_2-$, $-CH=CH(CH_2)_2-$, $-CH=CH(CH_2)_3-$ or $-CH_2C\equiv C(CH_2)_2-$.

Y may be for example $-CH_2-$, $-(CH_2)_2-$, $-(CH_2)_3-$, $-(CH_2)_4$, $-(CH_2)_5-$, $-CH_2CH=CH-$, $-CH_2C\equiv C-$, $-(CH_2)_2CH=CH-$ or $-(CH_2)_2C\equiv C-$.

Preferably the total number of carbon atoms in the chains X and Y is 6 to 12 inclusive and may be for example 7,8,9 or 10. Compounds wherein the sum total of carbon atoms in X and Y is 7,8 or 9 are particularly preferred.

A preferred group of compounds of formula (I) are those in which X and Y both represent an alkylene group within the meaning defined above.

In the definition of Q in compounds of formula (I), $R^{12}$ may be, for example, $-CH_2-$, $-\underset{CH_3}{CH}-$, $-(CH_2)_2-$ or $-(CH_2)_3-$. "Halogen" in the definition of $R^{13}$ of $R^{14}$ may be for example chlorine or fluorine. $R^{16}$, $R^{17}$, $R^{26}$ and $R^{27}$ may each be, for example, a hydrogen atom or a methyl, ethyl, propyl or isopropyl group. $R^{18}$ may be a methyl, ethyl, propyl or isopropyl group. $R^{19}$ may be for example a methyl, ethyl or propyl group.

Q in compounds of formula (I) may be for example

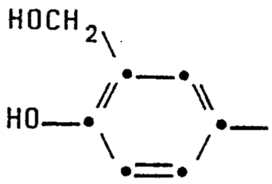, 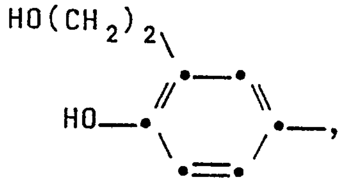, 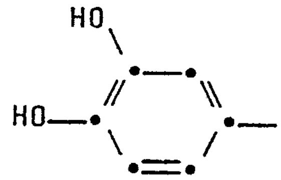, 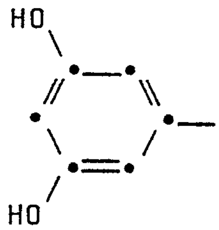, 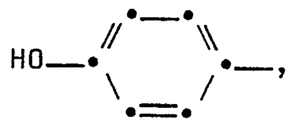, 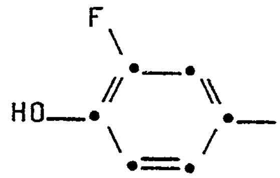, 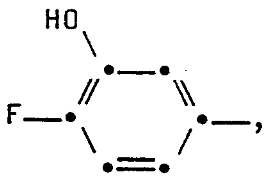, 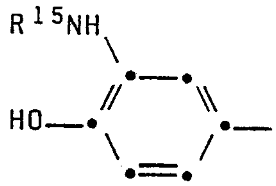 (where $R^{15}$ is HCO-, $CH_3CO-$, $NH_2CO-$, $(CH_3)_2NSO_2-$ or $CH_3SO_2-$), 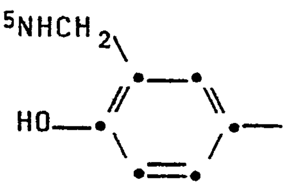 (where $R^{15}$ is as just defined), 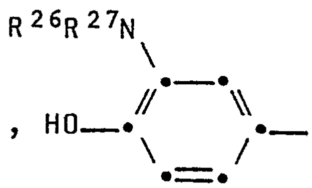 (where $R^{26}$ is hydrogen and $R^{27}$ is methyl),

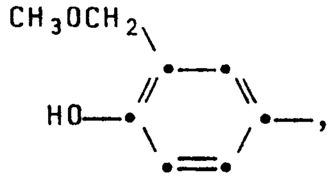, 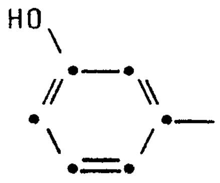 or a group of type (f), (g), (h) or (i).

Preferred meanings for the group Q include

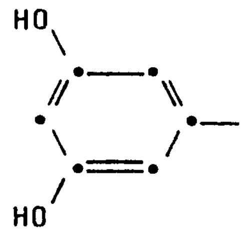 or more particularly 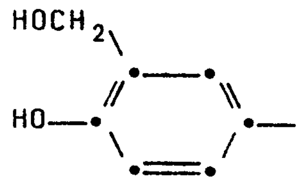 .

A preferred group of compounds according to the invention are those where in $R^1$ and $R^2$ represent hydrogen, $R^{20}$ represents a methyl or ethyl group, Ar represents a phenyl group, X represents the group $(CH_2)_5$, Y represents the group $(CH_2)_2$ or $(CH_2)_3$ and Q represents the group

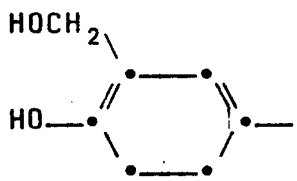 or 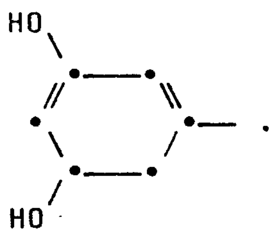 .

Suitable physiologically acceptable salts of the compounds of general formula (I) include acid addition salts derived from inorganic and organic acids, such as hydrochlorides, hydrobromides, sulphates, phosphates, maleates, tartrates, citrates, benzoates, 4-methoxybenzoates, 2- or 4-hydroxybenzoates, 4-chlorobenzoates, p-toluenesulphonates, methanesulphonates, sulphamates, ascorbates, salicylates, gluconates, tricarballylates, hydroxy-naphthalenecarboxylates e.g. 1-hydroxy- or 3-hydroxy-2-naphthalenecarboxylates, or oleates. The compounds may also form salts with suitable bases. Examples of such salts are alkali metal (e.g. sodium and potassium), and alkaline earth metal (e.g. calcium or magnesium) salts.

The compounds according to the invention have a stimulant action at $\beta_2$-adrenoreceptors, which furthermore is of a particularly advantageous profile. The stimulant action was demonstrated in the isolated trachea of the guinea-pig, where compounds were shown to cause relaxation of PGF2α-induced contractions. Compounds according to the invention have shown a particularly long duration of action in this test.

The compounds according to the invention may be used in the treatment of diseases associated with reversible airways obstruction such as asthma and chronic bronchitis.

The compounds according to the invention are also indicated as useful for the treatment of inflammatory and allergic skin diseases, congestive heart failure, depression, premature labour, glaucoma and in the treatment of conditions in which there is an advantage in lowering gastric acidity particularly in gastric and peptic ulceration.

The invention accordingly further provides compounds of formula (I) and their physiologically acceptable salts and solvates for use in the therapy or prophylaxis of diseases associated with reversible airways obstruction in human or animal subjects.

The compounds according to the invention may be formulated for administration in any convenient way. The invention therefore includes within its scope pharmaceutical compositions comprising at least one compound of formula (I) or a physiologically acceptable salt or solvate thereof formulated for use in human or veterinary medicine. Such compositions may be presented for use with physiologically acceptable carriers or excipients, optionally with supplementary medicinal agents.

The compounds may be formulated in a conventional manner on forms suitable for administration by inhalation or insufflation, or for oral, buccal, parenteral, topical (including nasal) or rectal administration.

Administration by inhalation or insufflation is preferred. The pharmaceutical compositions may be prepared by conventional means using physiologically acceptable excipients.

A proposed daily dosage of active compound for the treatment of man is 0.005mg to 100mg, which may be conveniently administered in one or two doses. The precise dose employed will of course depend on the age and condition of the patient and on the route of administration. Thus a suitable dose for administration by inhalation is 0.005mg to 20mg, for oral administration is 0.02mg to 100mg, and for parenteral administration is 0.01mg to 2mg for administration by bolus injection and 0.01 mg to 25mg for administration by infusion.

The compounds according to the invention may be prepared by a number of processes, as described in the following. In describing the processes which may be used for preparing compounds of formula (I) and intermediates useful in the preparation thereof, X, Y, $R^1$, $R^2$, $R^{20}$ and Ar are as defined for general formula (I) unless otherwise specified. In addition, any substituent in the group Ar may be a precursor substituent which is convertible into the rquired substituent by conventional methods.

It will be appreciated that certain of the reactions described below are capable of affecting other groups in the starting material which are desired in the end product; this applies especially in the reduction processes described, particularly where a hydride reducing agent is used and end-products are required containing the substituent $R^{16}CO$-. Care must therefore be taken in accordance with conventional practice, either to use reagents which will not effect such groups or to perform the reaction as part of a sequence which avoids their use when such groups are present in the starting material. In the preparation of both intermediates and end-product the final step in the reaction may be the removal of a protecting group. Suitable protecting groups and their removal are described in general process (2) below.

According to one general process (1), a compound of general formula (I) may be prepared by alkylation. Conventional alkylation procedures may be used.

Thus, for example, in one process (a), a compound of general formula (I) in which $R^1$ is a hydrogen atom may be prepared by alkylation of an amine of general formula (II)

```
        R20
        |
Q—CHCHNR22R23          (II)
  |
  OH
```

(wherein $R^{22}$ is a hydrogen atom or a protecting group, $R^{23}$ is a hydrogen atom, and any hydroxyl group(s) or amino group in the group Q may be protected) followed by removal of any protecting group where present.

The alkylation (a) may be effected using an alkylating agent of general formula (III):

```
L CH XOYAr (III)
  |
  R2
```

(wherein L represents a leaving group, for example a halogen atom such as chlorine, bromine or iodine or a hydrocarbylsulphonyloxy group such as methanesulphonyloxy or p-toluenesulphonyloxy).

The alkylation is preferably effected in the presence of a suitable acid scavenger, for example, inorganic bases such as sodium or potassium carbonate, organic bases such as triethylamine, diisopropylethylamine or pyridine, or alkylene oxides such as ethylene oxide or propylene oxide. The reaction is conveniently effected in a solvent such as acetonitrile or an ether e.g. tetrahydrofuran or dioxan, a ketone e.g. butanone or methyl isobutyl ketone, a substituted amide e.g. dimethylformamide or a chlorinated hydrocarbon e.g. chloroform at a temperature between ambient and the reflux temperature of the solvent.

According to another example (b) of an alkylation process, a compound of general formula (I) in which $R^1$ represents a hydrogen atom may be prepared by alkylation of an amine or general formula (II) as previously defined except that $R^{23}$ is a hydrogen atom or a group convertible thereto under the reaction conditions, with a compound of general formula (IV):

$R^2$COXOYAr (IV)

in the presence of a reducing agent, followed when necessary by removal of any protecting groups.

Examples of suitable $R^{23}$ groups convertible into a hydrogen atom are arylmethyl groups such as benzyl, α-methylbenzyl and benzhydryl.

Suitable reducing agents include hydrogen in the presence of a metal catalyst such as platinum, platinum oxide, palladium, palladium oxide, Raney nickel or rhodium, on a support such as charcoal, using an alcohol, e.g. ethanol or an ester e.g. ethyl acetate or an ether e.g. tetrahydrofuran, or water as reaction solvent, or a mixture of solvents, e.g. a mixture of two or more of those just described at normal or elevated temperature and pressure, for example from 20 to 100°C and from 1 to 10 atmospheres. Alternatively when one or both of $R^{22}$ and $R^{23}$ are hydrogen atoms, the reducing agent may be a hydride such as diborane or a metal hydride such as sodium borohydride, sodium cyanoborohydride or lithium aluminium hydride. Suitable solvents for the reaction with these reducing agents will depend on the particular hydride used, but will include alcohols such as methanol or ethanol, or ethers such as diethyl ether or tert-butyl methyl ether, or tetrahydrofuran.

When a compound of formula (II) where $R^{22}$ and $R^{23}$ are each hydrogen atoms is used, the intermediate imine of formula (V) may be formed:

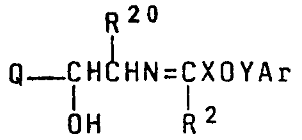 (V)

(where any hydroxyl or amino group(s) in Q may be protected).

Reduction of the imine using the conditions described above, followed, where necessary, by removal of any protecting groups, gives a compound of general formula (I).

Where it is desired to use a protected intermediate of general formula (II) it is particularly convenient to use hydrogen and a catalyst as described above with protecting group $R^{22}$ and any protecting group(s) in Q which are capable of being converted to a hydrogen atom under these reducing conditions, thus avoiding the need for a separate deprotection step. Suitable protecting groups of this type include arylmethyl groups such as benzyl, benzhydryl and α-methylbenzyl.

In another general process (2), a compound of general formula (I) may be obtained by deprotection of a protected intermediate of general formula (VI):

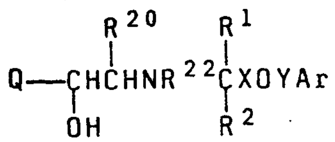 (VI)

(wherein $R^{22}$ and Q are as defined in formula (II) except that either $R^{22}$ is a protecting group and/or at least one of the hydroxyl or amino group(s) in Q is protected).

The protecting group may be any conventional protecting group, for example as described in "Protective Groups in Organic Chemistry", Ed. J.F.W. McOmie (Plenum Press, 1973). Examples of suitable hydroxyl protecting groups(s) within the group Q are arylmethyl groups such as benzyl, diphenylmethyl or triphenylmethyl and tetrahydropyranyl. Examples of suitable amino protecting groups represented by $R^{22}$ or used for the amino group $R^{26}R^{27}N$ within Q are arylmethyl groups such as benzyl, α-methylbenzyl, diphenylmethyl or triphenylmethyl and acyl groups such as trichloroacetyl or trifluoroacetyl.

The deprotection to yield a compound of general formula (I) may be effected using conventional techniques. Thus for example, an arylmethyl group may be cleaved by hydrogenolysis in the presence of the metal catalyst (e.g. palladium on charcoal). When a hydroxyl group is protected as tetrahydropyranyl this may be cleaved by hydrolysis under acidic conditions. Acyl groups may be removed by hydrolysis, for example with a base such as sodium hydroxide, or a group such as trichloroacetyl may be removed by reduction with, for example, zinc and acetic acid.

In a particular embodiment of the deprotection process (2), a compound of general formula (VII):

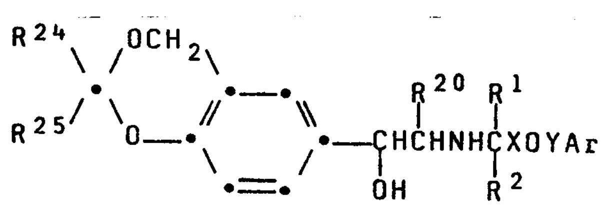 (VII)

(wherein $R^{24}$ and $R^{25}$, which may be the same or different, each represents a hydrogen atom or an alkyl or aryl group) may be deprotected.

A compound of general formula (I) may be obtained by treatment of a compound of formula (VII) with a dilute acid, for example hydrochloric acid in a solvent such as water or an alcohol such as ethanol at normal or elevated temperature.

In another general process (3), a compound of general formula (I) may be prepared by reduction. Thus, for example, a compound of general formula (I) may be prepared by reducing an intermediate of general formula (VIII):

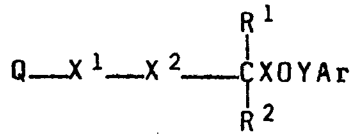 (VIII)

(wherein Q is as defined for general formula (II) and at least one of $X^1$ and $X^2$ represents a reducible group,

and/or Q contains a reducible group and the other(s) take the appropriate meaning as follows, which is $X^1$ is -CH(OH)-, $X^2$ is $-CHR^{20}NR^{22}-$ (where $R^{22}$ is hydrogen or a protecting group), and Q is as defined in formula (I), followed where necessary by removal of any protecting groups.

Suitable reducible groups include those wherein $X^1$ is a group $>C=O$, $X^2$ is a group $-CHR^{20}NY^1-$ (wherein $Y^1$ represents a group convertible to hydrogen by catalytic hydrogenation, for example an arylmethyl group such as benzyl, benzhydryl or α-methylbenzyl), and Q contains a substituent -CHO, $-CO_2R^{28}$ (where $R^{28}$ represents a hydrogen atom or an alkyl (e.g. $C_{1-3}$ alkyl) group or $-NR^{26}R^{29}$ where $R^{29}$ is an alkoxycarbonyl, aralkyloxycarbonyl, $C_{2-4}$alkanoyl or formyl group).

In one convenient aspect of the reduction process, any hydroxyl or amino group(s) within Q may be protected as a group which is convertible to hydrogen under the reducing conditions employed and may be for example an arylmethyl group such as benzyl, benzhydryl or α-methylbenzyl.

The reduction may be effected using for example reducing agents conveniently employed for the reduction of carboxylic acids, aldehydes, esters, ketones and protected amines.

Thus for example when $X^1$ in general formula (VIII) represents a $>C=O$ group this may be reduced to a -CH(OH)-group using hydrogen in the presence of a catalyst as previously described for process (1) part (b). Alternatively, the reducing agent may be, for example, a hydride such as diborane or a metal hydride such as lithium aluminium hydride, sodium bis(2-methoxyethoxy) aluminium hydride (Red-Al), sodium borohydride or aluminium hydride. The reaction may be effected in a solvent, where appropriate an alcohol e.g. methanol or ethanol, or an ether such as tetrahydrofuran, or a halogenated hydrocarbon such as dichloromethane, at a temperature of 0°C to the refux temperature of the solvent.

When $X^2$ in general formula (VIII) represents a $-CHR^{20}NY^1-$ group this may be reduced to a $-CHR^{20}NH-$ group using hydrogen in the presence of a metal catalyst as previously described for process (1) part (b).

When Q in general formula (VIII) contains a substituent -CHO or $-CO_2R^{28}$ this may be reduced to a group $-CH_2OH$ using a hydride or complex metal hydride reducing agent as described above for the reduction of $>C=O$ to -CH(OH)-. When Q in general formula (I) contains a substituent $-NR^{26}R^{29}$ this may be reduced to a group $-NR^{26}R^{27}$ where $R^{27}$ represents an alkyl group using for example a complex metal hydride e.g. lithium aluminium hydride in a solvent such as an ether e.g. tetrahydrofuran.

In the general processes described above, the compound of formula (I) obtained may be in the form of a salt, conveniently in the form of a physiologically acceptable salt. Where desired, such salts may be converted to the corresponding free acids using conventional methods.

Physiologically acceptable salts of the compounds of general formula (I) may be prepared by reacting a compound of general formula (I) with an appropriate acid or base in the presence of a suitable solvent such as acetonitrile, acetone, chloroform, ethyl acetate or an alcohol, e.g. methanol, ethanol or iso-propanol.

Physiologically acceptable salts may also be prepared from other salts, including other physiologically acceptable salts, of the compounds of general formula (I), using conventional methods.

When a specific enantiomer of a compound of general formula (I) is required, this may be obtained by resolution of a corresponding racemate of a compound of general formula (I) using conventional methods.

Thus, in one example an appropriate optically active acid may be used to form salts with the racemate of a compound of general formula (I). The resulting mixture of isomeric salts may be separated for example by fractional crystallisation, into the diastereoisomeric salts from which the required enantiomer of a compound of general formula (I) may be isolated by conversion into the required free base.

Alternatively, enantiomers of a compound of general formula (I) may be synthesised from the appropriate optically active intermediates using any of the general processes described herein.

Specific diastereoisomers of a compound of formula (I) may be obtained by conventional methods for example, by synthesis from an appropriate asymmetric starting material using any of the processes described herein, or by conversion of a mixture of isomers of a compound of general formula (I) into appropriate diastereoisomeric derivatives e.g. salts which then can be separated by conventional means e.g. by fractional crystallisation.

Intermediate compounds of general formula (VIII) for use in general process (3) may be prepared by a number of processes.

Thus for example intermediates of general formula (VIII) in which $X^1$ is a group $>C=O$ may be prepared from a haloketone of formula (IX):

$Q-COCHR^{20}Hal$ (IX)

(wherein any hydroxyl or amino group(s) in the group Q may optinally be protected) by reaction with an amine of general formula (X)

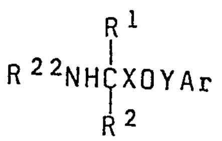

(X)

(wherein $R^{22}$ is a hydrogen atom or a group convertible thereto by catalytic hydrogenation).

The reaction may be effected in a cold or hot solvent, for example tetrahydrofuran, tert-butyl methyl ether,

dioxan, chloroform, dimethylformamide, acetonitrile or a ketone such as butanone or methylisobutylketone, or an ester, for example ethyl acetate preferably in the presence of a base such as diisopropylethylamine, sodium carbonate or other acid scavenger such as propylene oxide.

Intermediates of general formula (VIII) in which $X^1$ is a group $>C=O$ may be reduced to the corresponding intermediate in which $X^1$ is a group -CH(OH)- using for example metal hydride such as sodium borohydride in a solvent e.g. ethanol.

Amines of formula (II), haloketones of formula (IX), and intermediates of formulae (III), (IV) and (X) are either known compounds or may be prepared by methods analogous to those described for the preparation of known compounds.

Suitable methods for preparing intermediates of formulae (III), (IV) and (X) are described in UK Patent Specification No. 2140800A and in the exemplification included hereinafter.

The following examples illustrate the invention. Temperatures are in °C. 'Dried' refers to drying using magnesium sulphate except where otherwise stated. Thin layer chromatography (T.l.c.) was carried out over $SiO_2$. Flash column chromatography (FCC) was carried out using silica (Merck 9385) unless otherwise stated. The following abbreviations are used: DMF - dimethylformamide; THF -tetrahydrofuran; EA -ethyl acetate; ER - diethyl ether.

Intermediate 1

1-[3,5-Bis(phenylmethoxy)phenyl]-2-[[6-(3-phenylpropoxy)hexyl](phenylmethyl) amino]-1-propanone

A mixture of Intermediate 8 (425mg), N-[6-(3-phenylpropoxy)hexyl]benzenemethanamine hydrobromide (423mg) and N,N-diisopropylethylamine (284mg) in DMF (5mℓ) was stirred under nitrogen for 18h at room temperature and at 70° for 4h. The solution was cooled, poured into water (50mℓ) and extracted with EA (2x25mℓ). The organic phase was washed with water (50mℓ), dried ($Na_2SO_4$) and evaporated onto FCC silica. The impregnated material was applied to a column of FCC silica, elution with EA-hexane (1:6) yielding the title compound as a viscous yellow oil (475mg). T.l.c. (EA-hexane 1:4) Rf 0.53.

Intermediate 2

N-[6-(3-Phenylpropoxy)hexyl]benzenemethanamine hydrobromide

Intermediate 3

erythro-Methyl 2-hydroxy-5-[1-hydroxy-2-[[6-(3-phenylpropoxy)hexyl]amino]propyl] benzoate

A mixture of methyl 5-(2-bromopropionyl)-2-hydroxybenzoate (0.96g), Intermediate 2 (1.51g) and N,N-diisopropylethylamine (0.89g) in dichloromethane (25ml) was stirred and heated under reflux under nitrogen for 48h. The solution was evaporated on to silica (Art 9385: ca. 10g) and the impregnated material applied to a 'flash' chromatography column, (Merck 9385: 20 x 3.5 cm) and eluted with ER/cyclohexane (1:3). A solution of the resulting oil (1.0g) in absolute ethanol (35ml) was hydrogenated at room temperature and atmospheric pressure over a pre-reduced 10% palladium oxide on carbon (0.1g) and 5% platinum oxide on carbon (0.1g) catalyst. The catalyst was removed by filtration through 'hyflo' and the solvent removed in vacuo at 40° to yield the crude product as a pale yellow oil which was purified by FCC (using triethylamine-deactivated Merck 9385 silica), eluting with EA/methanol (8:1) to give the title compound as a colourless oil (0.36g). T.l.c. (Toluene-EtOH-0.88$NH_3$ 39:10:1) Rf 0.47.

Intermediate 4

N-[6-(2-Phenylethoxy)hexyl]benzenemethanamine

Intermediate 5

6-(2-Phenylethoxy)hexanamine

Intermediate 4 (7.1g) in ethanol (100mℓ) was hydrogenated over pre-reduced 10% palladium on charcoal (50% paste in water, 0.7g). The reaction mixture was filtered (hyflo) and the filtrate evaporated to give the title compound as a colourless oil (4.92g). T.l.c. EA + few drops $Et_3N$) Rf 0.1.

Intermediate 6

Methyl 2-hydroxy-5-[1-oxo-2-[[6-(2-phenylethoxy)hexyl] amino]butyl]benzoate

A solution of methyl-5-[2-bromo-1-oxobutyl)-2-hydroxybenzoate (6.67g), Intermediate 5 (4.9g) and N,N-diisopropylethylamine (6.5g) in methanol (100mℓ) was stirred at reflux, under nitrogen for 24h. The methanol was evaporated, the residue was dissolved in EA (100mℓ) washed with water and brine, dried ($Na_2SO_4$) and concentrated to an oil. The oil was purified by FCC eluting with cyclohexane-EA-triethylamine (66:33:1)→EA-triethylamine (99:1) to give the title compound as an orange oil (35mg). T.l.c. (EA-triethylamine

99:1) Rf 0.41.

Intermediate 7

erythro-Methyl 2-hydroxy-5-[1-hydroxy-2-[[6-(2-phenylethoxy)hexyl]amino]butyl]benzoate

Intermediate 6 (3.0g) was hydrogenated in ethanol (60mℓ) over 5% platinum on carbon (500mg). The catalyst was removed by filtration through hyflo and the ethanol was evaporated. The residual oil was purified by FCC eluting with hexane-EA (7:3) to give a pale yellow oil, which was further hydrogenated for 4h using the same conditions as above. After filtration and evaporation the title compound was obtained as an oil (2.05g). T.l.c. (EA-triethylamine 99:1) Rf 0.40.

Intermediate 8

2-Bromo-1-[3,5-bis(phenylmethoxy)phenyl]-1-propanone

(i) 3,5-Bis(phenylmethoxy)benzaldehyde, oxime

A mixture of 3,5-bis(phenylmethoxy)benzaldehyde (31.8g), anhydrous sodium acetate (27.1g) and hydroxylamine hydrochloride (27.8g) was stirred and heated under reflux in absolute ethanol (400mℓ) for 2h. The solvent was removed in vacuo at 40° and the residue partitioned between EA (500mℓ) and water (500mℓ). The organic phase was washed with water (250mℓ), dried and concentrated to afford the title compound as a cream solid (32.4g). A portion (1g) was recrystallised from EA-hexane affording a colourless crystalline powder m.p. 113-116°.

(ii) 3,5-Bis(phenylmethoxy)benzonitrile

A solution of the product of stage (i) (30.0g) in acetic anhydride (400mℓ) was stirred and heated under reflux under nitrogen for 6h. The acetic anhydride was then removed under high vacuum at 50° yielding a fawn solid which was recrystallised from absolute ethanol (ca 175mℓ) to afford the title compound as a cream crystalline powder (22.8g) m.p. 108-110°.

(iii) 1-[3,5-Bis(phenylmethoxy)phenyl]-1-propanone

A solution of ethylmagnesium bromide was prepared by treating magnesium turnings (2.19g) in dry ether (10mℓ) with a solution of ethyl bromide (9.81g) in dry ether (90mℓ) under nitrogen. When all the magnesium had been consumed, the product of stage (ii) (9.45g) in a mixture of dry ether (50mℓ) and THF (50mℓ) was added dropwise over 15min, and then the mixture stirred and heated under reflux under nitrogen for 2h. The solution was cooled and 2N hydrochloric acid (10mℓ) added dropwise with caution, then further 2N HCl (200mℓ) introduced. The two-phase mixture was vigorously stirred for 2h, the phases were separated, the aqueous phase extracted with ether (50mℓ) and the organic solutions combined, dried and treated with charcoal. Concentration yielded the title compound as a cream crystalline solid (9.6g) which was recrystallised from absolute ethanol affording a colourless crystalline powder (8.5g) m.p. 74-75°.

(iv) 2-Bromo-1-[3,5-bis(phenylmethoxy)phenyl]-1-propanone

Bromine (2.13g) in chloroform (25mℓ) was added dropwise to a stirred solution of the product of stage (iii) (4.62g) in ether (125mℓ) at such a rate so as to maintain a colourless solution. When the addition was complete, propylene oxide (1mℓ) was added, the mixture stirred for 5 min and the solvents evaporated in vacuo at 40°. The product was purified by FCC eluting with EA-hexane (1:4) to afford the title compound as a colourless oil which solidified to a waxy solid (5.31g) on standing. This was recrystallised from warm methanol yielding a colourless crystalline powder (4.2g) m.p. 65-67°.

Example 1

erythro-4-Hydroxy-$\alpha^1$-[1-[[6-(3-phenylpropoxy)hexyl]amino] ethyl]1,3-benzenedimethanol hydrochloride

A solution of Intermediate 3 (0.35g) in THF (10ml) was added dropwise to a stirred suspension of lithium aluminium hydride (0.15g) in THF (5ml) and the mixture stirred and heated under reflux under nitrogen for 2.5h. 2N Hydrochloric acid (5ml) was cautiously added, the majority of the solvent removed in vacuo at 35° and the residual oil diluted with further 2N hydrochloric acid then extracted with EA (2x25ml). The organic phase was washed with 8% sodium bicarbonate solution (50ml), dried ($Na_2SO_4$) and concentrated to yield the product as a pale yellow gum. A solution of the latter in EA (5ml) was treated with an excess of ethereal hydrogen chloride and diluted with ER (25ml) to afford the title compound as an off-white powder (135mg) m.p. 85-86°. T.l.c. (Toluene-EtOH-0.88$NH_3$ 339:10:1) Rf 0.25.

Example 2

erythro-4-Hydroxy-α[1]-[1-[[6-(2-phenylethoxy)hexyl] amino]propyl]-1,3-benzene dimethanol hydrochloride

A solution of Intermediate 8 (1.0g) in dry toluene (5mℓ) was added over 10 min to a stirred solution of Red-Al (3.4M in toluene, 2.1mℓ) in dry toluene (10mℓ) at -10° under nitrogen. The solution was allowed to warm to room temperature and stirred for 1h, cooled in an ice-bath and treated with water (1.2mℓ). Water (10mℓ) and 2N hydrochloric acid (20mℓ) were added and the mixture was extracted with EA (3x50mℓ). The extracts were evaporated and the resultant wet gum was dried by azeotroping with toluene. Trituration with ER afforded a white powder (200mg) which was purified by FCC eluting with EA-methanol (9:1) to give the title compound as a white powder (50mg) m.p. 110-111°. T.l.c. (EA-methanol 9:1) Rf 0.20.

Example 3

erythro-5-[1-Hydroxy-2-[[6-(3-phenylpropoxy)hexyl]amino] propyl]-1,3-benzenediol, hydrochloride

A solution of Intermediate 1 (430mg) in a mixture of absolute ethanol (15mℓ) and THF (5mℓ) was hydrogenated at room temperature and atmospheric pressure over a pre-reduced 5% $PtO_2$ on carbon (50mg) and 10% PdO on carbon (100mg, 50% paste in water) catalyst mixture in absolute ethanol (5mℓ). The catalyst was removed by filtration through 'hyflo', the solution treated with an excess of ethereal hydrogen chloride and the solvents evaporated in vacuo at 40° to afford the title compound as an off-white foam (193mg) m.p. 42-48°. T.l.c. (Toluene:EtOH:0.88$NH_4OH$ 39:10:1) Rf 0.56.

Example 4

The following are examples of suitable formulations of compounds of the invention. The term "active ingredient" is used herein to represent a compound of the invention.

Tablets (Direct Compression)

| | mg/tablet |
|---|---|
| Active ingredient | 2.0 |
| Microcrystalline Cellulose USP | 196.5 |
| Magnesium Stearate BP | 1.5 |
| Compression weight | 200.0 |

The active ingredient in sieved through a suitable sieve, blend with the excipients and compressed using 7mm diameter punches.

Tablets of other strengths may be prepared by altering the ratio of active ingredient to microcrystalline cellulose or the compression weight and using punches to suit.

The tablets may be film coated with suitable film forming materials, such as hydroxypropylmethylcellulose, using standard techniques. Alternatively the tablets may be sugar coated.

Metered Dose Pressurised Aerosol (Suspension Aerosol)

| | mg/metered dose | Per can |
|---|---|---|
| Active ingredient micronised | 0.100 | 26.40mg |
| Oleic Acid BP | 0.100 | 2.64mg |
| Trichlorofluoromethane BP | 23.64 | 5.67g |
| Dichlorodifluoromethane BP | 61.25 | 14.70g |

The active ingredient is micronised in a fluid energy mill to a fine particle size range. The oleic acid is mixed with the trichlorofluoromethane at a temperature of 10-15°C and the micronise drug is mixed into the solution with a high shear mixer. The suspension is metered into aluminium aerosol cans and suitable metering valves delivering 85mg of suspension are crimped onto the cans and the dichlorodifluoromethane is pressure filled into the can through the valves.

Inhalation Cartridges

| | | mg/cartridge |
|---|---|---|
| Active ingredient micronised | | 0.200 |
| Lactose BP | to | 25.0 |

The active ingredient is micronised in a fluid energy mill to a fine particle size range prior to blending with normal tabletting grade lactose in a high energy mixer. The powder blend is filler in No. 3 hard gelatin capsules on a suitable encapsulating machine. Th contents of the cartridges are administered using a powder inhaler such as the Glaxo Rotahaler.

## Claims

1. Compounds of the general formula (I)

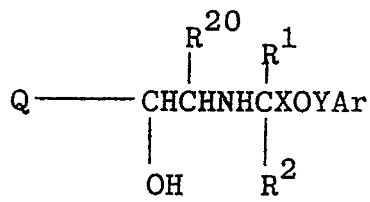

(I)

wherein
Ar represents a phenyl group optionally substituted by one or more substituents selected from halogen atoms, or the groups $C_{1-6}$ alkyl, nitro, $-(CH_2)_qR$, [where R is hydroxy, $C_{1-6}$ alkoxy, $-NR^3R^4$ (where $R^3$ and $R^4$ each represents a hydrogen atom, or a $C_{1-4}$ alkyl group, or $-NR^3R^4$ forms a saturated heterocyclic amino group which has 5-7 ring members and optionally contains in the ring one or more atoms selected from -O- or -S- or a group -NH- or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$alkoxy or $-NR^3R^4$), $-SR^9$ (where $R^9$ is a hydrogen atom, or a $C_{1-4}$ alkyl or phenyl group), $-SOR^9$, $-SO_2R^9$, or -CN, and q represents an integer from O to 3], or $-O(CH_2)_tR^{11}$ [where $R^{11}$ represents a hydroxy or $C_{1-4}$ alkoxy group and t is 2 or 3], or Ar is a phenyl group substituted by an alkylenedioxy group of formula $-O(CH_2)_pO-$, where p represents 1 or 2;
$R^1$ and $R^2$ each represent a hydrogen atom or a $C_{1-3}$ alkyl group, with the proviso that the sum total of carbon atoms in $R^1$ and $R^2$ is not more than 4;
$R^{20}$ represents a $C_{1-3}$ alkyl group;
X represents a $C_{2-8}$ alkylene, $C_{2-8}$ alkenylene or $C_{2-8}$alkynylene chain, and
Y represents a $C_{1-7}$ alkylene, $C_{2-7}$ alkenylene or $C_{2-7}$alkynylene chain, with the proviso that the sum total of carbon atoms in X and Y is 4 to 12;
Q represents (a)

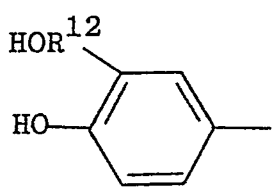

(where $R^{12}$ is a straight or branched $C_{1-3}$ alkylene group),

(b)

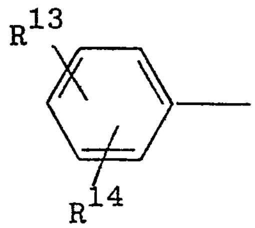

(where one of $R^{13}$ and $R^{14}$ is a hydroxy group and the other is a hydrogen or halogen atom or a hydroxy group),

(c) 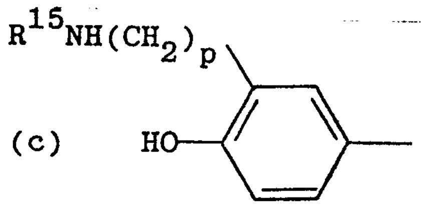

[where $R^{15}$ is a group $R^{16}CO$-, $R^{16}NHCO$-, $R^{16}R^{17}NSO_2$- or $R^{18}SO_2$- (where $R^{16}$ and $R^{17}$ is each a hydrogen atom or a $C_{1-3}$ alkyl group and $R^{18}$ is a $C_{1-3}$ alkyl group) and p is an integer 0 or 1],

(d) 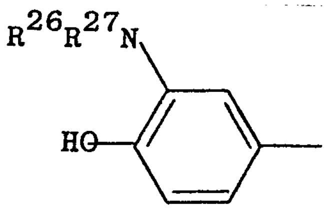

(where $R^{26}$ and $R^{27}$ is each a hydrogen atom or a $C_{1-3}$ alkyl group or, when $R^{26}$ is a hydrogen atom, $R^{27}$ may also be a $C_{1-4}$ alkoxycarbonyl group),

(e) 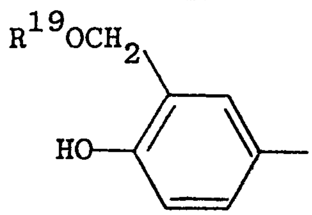,

(where $R^{19}$ is a $C_{1-3}$ alkyl group),

(f) 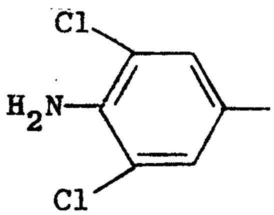

(g) 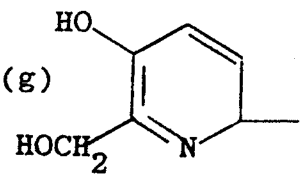

(h) 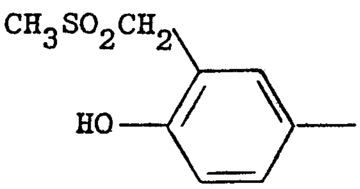 or (i) 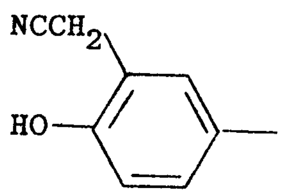

and physiologically acceptable salts and solvates thereof.

2. Compounds as claimed in claim 1 in which $R^1$ is a hydrogen atom or a methyl group, and $R^2$ is a hydrogen atom.

3. Compounds as claimed in claim 1 or 2 in which $R^{20}$ is a methyl or ethyl group.

4. Compounds as claimed in any of claims 1 to 3 in which X is a $C_{2-8}$ alkylene chain and Y is a $C_{1-7}$ alkylene chain.

5. Compounds as claimed in any of claims 1 to 4 in which the sum total of carbon atoms in X and Y is 7, 8 or 9.

6. Compounds as claimed in any of claims 1 to 5 in which Ar is phenyl; or Ar is phenyl substituted by one or more substituents selected from chlorine, bromine, iodine, fluorine, methyl, ethyl, ethoxy, $-(CH_2)_q$ R [where R is hydroxy, methoxy, amino, methylamino, ethylamino, dimethylamino, diethylamino, morpholino, piperidino, piperazino, N-methylpiperazino, $-NHCOR^6$ (where $R^6$ is hydrogen or $C_{1-4}$ alkyl,

$C_{1-4}$ alkoxy, phenyl, amino or N,N-dimethylamino), $-N(CH_3)COCH_3$, $-NR^5SO_2R^7$, (where $R^5$ represents a hydrogen atom or a methyl group and $R^7$ represents methyl, butyl, phenyl, amino or dimethylamino), -COOH, $-COOCH_3$, $-COOCH_2CH_2CH_3$, $-CONH_2$, $-CON(CH_3)_2$, $-CON(CH_2CH_3)_2$, $-CON(CH_2CH_2CH_3)_2$, $-SR^9$ (where $R^9$ is methyl, ethyl or phenyl), $-SOCH_3$, $-SO_2CH_3$, or CN, and q is zero, 1, 2 or 3], $-NO_2$, $-O(CH_2)_2OH$, $-O(CH_2)_3OH$, $-O(CH_2)_2OCH_3$, or $-O(CH_2)_2OCH_2CH_3$.

7. Compounds as claimed in any of claims 1 to 6 in which Q represents

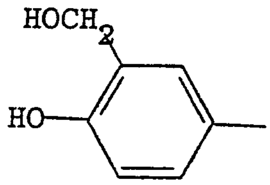 , 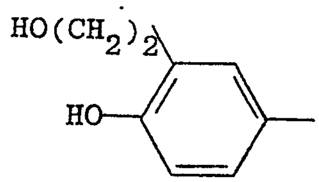 , 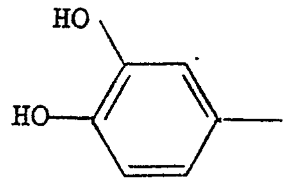 ,

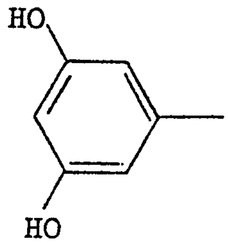 , 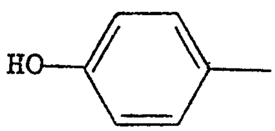 , 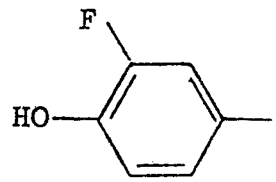 ,

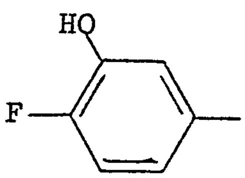 , 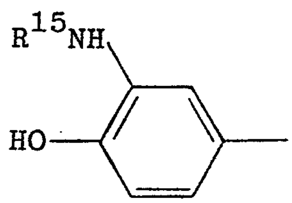 (where $R^{15}$ is HCO-,

(where $R^{15}$ is HCO-, $CH_3CO-$, $NH_2CO-$, $(CH_3)_2NSO_2-$ or $CH_3SO_2-$),

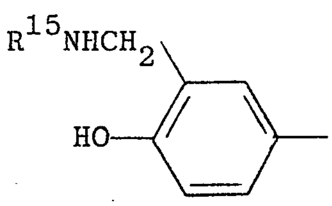

(where $R^{15}$ is as just defined),

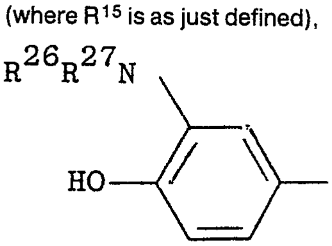 , (where

(where $R^{26}$ is hydrogen and $R^{27}$ is methyl),

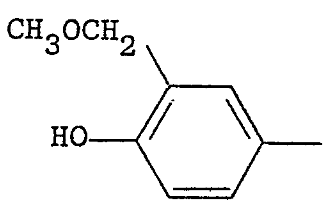 , 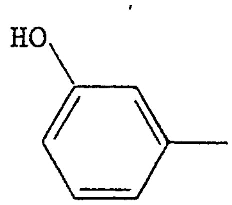

or a group of type (f), (g), (h), or (i).

8. Compounds as claimed in any of claims 1 to 7 in which $R^1$ and $R^2$ each represent a hydrogen atom, $R^{20}$ is a methyl or ethyl group, Ar is a phenyl group, X is the group $-(CH_2)_5-$, Y is the group $-(CH_2)_2-$ or $-(CH_2)_3-$ and Q is the group

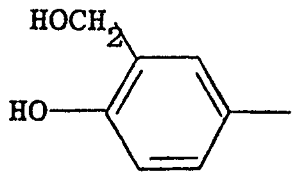

or

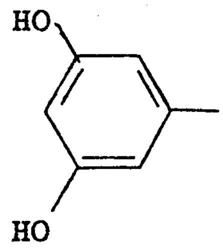

9. A process for the preparation of compounds as claimed in any of claims 1 to 8 or a physiologically acceptable salt or solvate thereof which comprises:

(1a) for the preparation of a compound of formula (I) in which $R^1$ is a hydrogen atom, alkylating an amine of general formula (II)

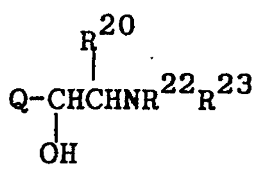

(II)

(wherein $R^{22}$ is a hydrogen atom or a protecting group, $R^{23}$ is a hydrogen atom, and any hydroxyl group(s) or amino group in the group Q may be protected) with an alkylating agent of general formula (III)

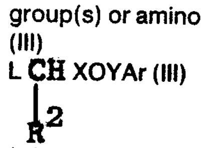

(III)

(wherein L represents a leaving group) followed, if necessary, by removal of any protecting group present; or

(1b) for the preparation of a compound of formula (I) in which $R^1$ is a hydrogen atom, alkylating an amine of general formula (II) as defined above axcept that $R^{23}$ is a hydrogen atom or a group convertible thereto under the reaction conditions, with a compound of general formula (IV)

$R^2COXCOYAr$ (IV)

in the presence of a reducing agent followed, if necessary, by removal of any protecting groups present; or

(2) deprotecting a protected intermediate of general formula (VI)

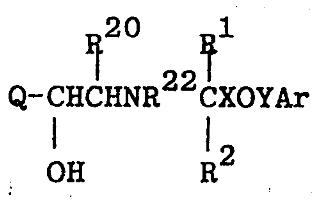

(VI)

wherein $R^{22}$ and Q are as defined in claim 1 except that either $R^{22}$ is a protecting group and/or at least one of the hydroxyl or amino groups in Q is protected); or

(3) reducing an intermediate of general formula (VIII)

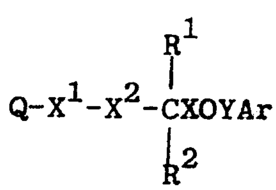

(VIII)

wherein Q is as defined in claim 1;

$X^1$ is -CH(OH) or a group convertible thereto by reduction;

$X^2$ is $-CHR^{20}NR^{22}-$ (where $R^{22}$ is hydrogen or a protecting group) or a group convertible thereto by reduction;

at least one of $X^1$ and $X^2$ representing a reducible group and/or Q containing a reducible group, followed, if necessary, by removal of any protecting group present; and

if desired, converting the resulting compound of general formula (I) or a salt thereof into a physiologically acceptable salt of solvate thereof.

10. A pharmaceutical composition comprising at least one compound of general formula (I) as defined in any of claims 1 to 8 or a physiologically acceptable salt or solvate thereof, together with a physiologically acceptable carrier or excipient.

Claims for the following Contracting States : AT, GR, ES

1. A process for the preparation of compounds of the general formula (I)

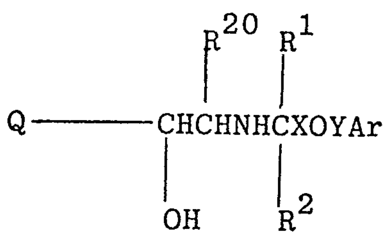

(I)

wherein

Ar represents a phenyl group optionally substituted by one or more substituents selected from halogen atoms, or the groups $C_{1-6}$ alkyl, nitro, $-(CH_2)_qR$, [where R is hydroxy, $C_{1-6}$ alkoxy, $-NR^3R^4$ (where $R^3$ and $R^4$ each represents a hydrogen atom, or a $C_{1-4}$ alkyl group, or $-NR^3R^4$ forms a saturated heterocyclic amino group which has 5-7 ring members and optionally contains in the ring one or more atoms selected from -O- or -S- or a group -NH- or $-N(CH_3)-$), $-NR^5COR^6$ (where $R^5$ represents a hydrogen atom or a $C_{1-4}$ alkyl group, and $R^6$ represents a hydrogen atom or a $C_{1-4}$ alkyl, $C_{1-4}$ alkoxy, phenyl or $-NR^3R^4$ group), $-NR^5SO_2R^7$ (where $R^7$ represents a $C_{1-4}$ alkyl, phenyl or $-NR^3R^4$ group), $-COR^8$ (where $R^8$ represents hydroxy, $C_{1-4}$alkoxy or $-NR^3R^4$), $-SR^9$ (where $R^9$ is a hydrogen atom, or a $C_{1-4}$ alkyl or phenyl group), $-SOR^9$, $-SO_2R^9$, or -CN, and q represents an integer from O to 3], or $-O(CH_2)_tR^{11}$ [where $R^{11}$ represents a hydroxy or $C_{1-4}$ alkoxy group and t is 2 or 3], or Ar is a phenyl group substituted by an alkylenedioxy group of formula $-O(CH_2)_pO-$, where p represents 1 or 2;

$R^1$ and $R^2$ each represents a hydrogen atom or a $C_{1-3}$ alkyl group, with the proviso that the sum total of carbon atoms in $R^1$ and $R^2$ is not more than 4;

$R^{20}$ represents a $C_{1-3}$ alkyl group;

X represents a $C_{2-8}$ alkylene, $C_{2-8}$ alkenylene or $C_{2-8}$alkynylene chain, and

Y represents a $C_{1-7}$ alkylene, $C_{2-7}$ alkenylene or $C_{2-7}$alkynylene chain, with the proviso that the sum total of carbon atoms in X and Y is 4 to 12;

Q represents (a)

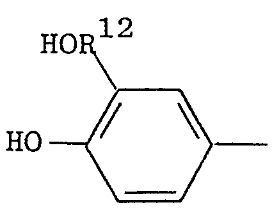

(where $R^{12}$ is a straight or branched $C_{1-3}$ alkylene group),

(b)

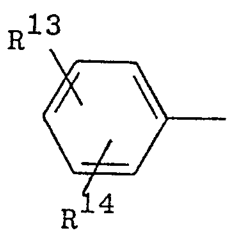

(where one of $R^{13}$ and $R^{14}$ is a hydroxy group and the other is a hydrogen or halogen atom or a hydroxy group),

(c) 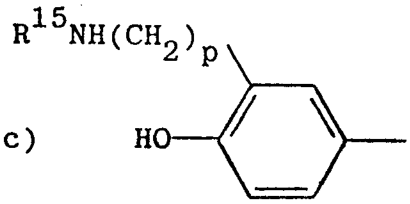

[where $R^{15}$ is a group $R^{16}CO$-, $R^{16}NHCO$-, $R^{16}R^{17}NSO_2$- or $R^{18}SO_2$- (where $R^{16}$ and $R^{17}$ is each a hydrogen atom or a $C_{1-3}$ alkyl group and $R^{18}$ is a $C_{1-3}$ alkyl group) and p is an integer 0 or 1],

(d) 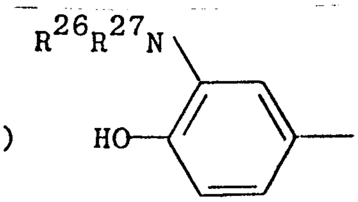

(where $R^{26}$ and $R^{27}$ is each a hydrogen atom or a $C_{1-3}$ alkyl group or, when $R^{26}$ is a hydrogen atom, $R^{27}$ may also be a $C_{1-4}$ alkoxycarbonyl group),

(e) 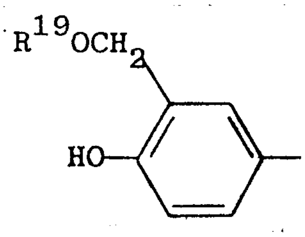

(where $R^{19}$ is a $C_{1-3}$ alkyl group),

(f) 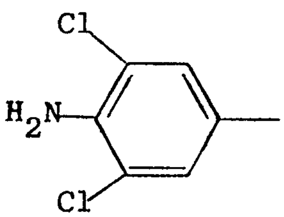

(g) 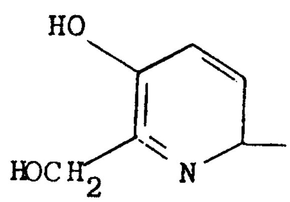

(h) 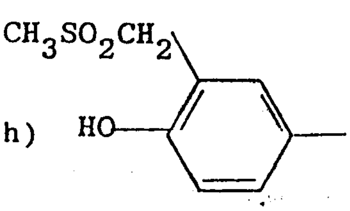 or (i) 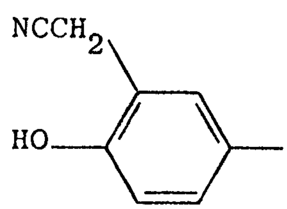

and physiologically acceptable salts and solvates thereof which comprises:

(1a) for the preparation of a compound of formula (I) in which $R^1$ is a hydrogen atom, alkylating an amine of general formula (II)

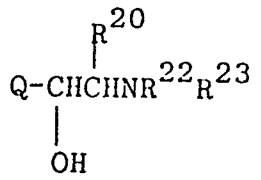

(II)

(wherein $R^{22}$ is a hydrogen atom or a protecting group, $R^{23}$ is a hydrogen atom, and any hydroxyl group(s) or amino group in the group Q may be protected) with an alkylating agent of general formula (III)

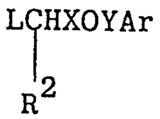

(III)

(wherein L represents a leaving group) followed, if necessary, by removal of any protecting group present; or

(1b) for the preparation of a compound of formula (I) in which $R^1$ is a hydrogen atom, alkylating an amine of general formula (II) as defined above except that $R^{23}$ is a hydrogen atom or a group convertible thereto under the reaction conditions, with a compound of general formula (IV)

$R^2COXOYAr$ (IV)

in the presence of a reducing agent followed, if necessary, by removal of any protecting groups present; or

(2) deprotecting a protected intermediate of general formula (VI)

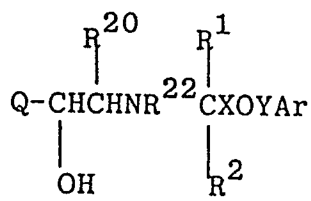

(VI)

(wherein $R^{22}$ and Q are as defined above except that either $R^{22}$ is a protecting group and/or at least one of the hydroxyl or amino groups in Q is protected); or

(3) reducing an intermediate of general formula (VIII)

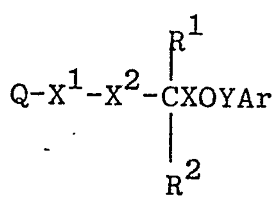

(VIII)

wherein Q is as defined above;

$X^1$ is -CH(OH) or a group convertible thereto by reduction;

$X^2$ is $-CHR^{20}NR^{22}-$ (where $R^{22}$ is hydrogen or a protecting group) or a group convertible thereto by redection;

at least one of $X^1$ and $X^2$ representing a reducible group and/or Q containing a reducible group; followed, if necessary, by removal of any protecting group present; and if desired, converting the resulting compound of general formula (I) or a salt thereof into a physiologically acceptable salt or solvate thereof.

2. A process as claimed in claim 1 for the production of compounds in which $R^1$ is a hydrogen atom or a methyl group, and $R^2$ is a hydrogen atom.

3. A process as claimed in claim 1 or 2 for the production of compounds in which $R^{20}$ is a methyl or ethyl group.

4. A process as claimed in any of claims 1 to 3 for the production of compounds in which X is a $C_{2-8}$ alkylene chain and Y is a $C_{1-7}$ alkylene chain.

5. A process as claimed in any of claims 1 to 4 for the production of compounds in which the sum total of carbon atoms in X and Y is 7, 8 or 9.

6. A process as claimed in any of claims 1 to 5 for the production of compounds in which Ar is phenyl; or Ar is phenyl substituted by one or more substituents selected from chlorine, bromine, iodine, fluorine, methyl, ethyl, ethoxy, $-(CH_2)_q$ R [where R is hydroxy, methoxy, amino, methylamino, ethylamino, dimethylamino, diethylamino, morpholino, piperidino, piperazino, N-methylpiperazino, $-NHCOR^6$ (where $R^6$ is hydrogen or $C_{1-4}$ alkyl, $C_{1-4}$ alkoxy, phenyl, amino or N,N-dimethylamino), $-N(CH_3)COCH_3$, $-NR^5SO_2R^7$, (where $R^5$ represents a hydrogen atom or a methyl group and $R^7$ represents methyl, butyl, phenyl, amino or dimethylamino), -COOH, $-COOCH_3$, $-COOCH_2CH_2CH_3$, $-CONH_2$, $-CON(CH_3)_2$, $-CON(CH_2CH_3)_2$, $-CON(CH_2CH_2CH_3)_2$, $-SR^9$ (where $R^9$ is methyl, ethyl or phenyl), $-SOCH_3$, $-SO_2CH_3$, and q is zero, 1, 2 or 3], $-NO_2$, $-O(CH_2)_2OH$, $-O(CH_2)_3OH$, $-O(CH_2)_2OCH_3$, or $-O(CH_2)_2OCH_2CH_3$.

7. A process as claimed in any of claims 1 to 6 for the production of compounds in which Q represents

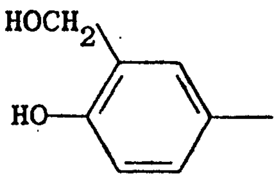 , 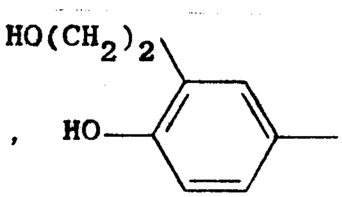 , 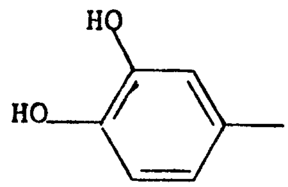 ,

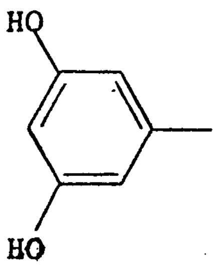 , 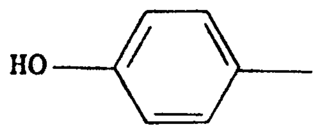 , 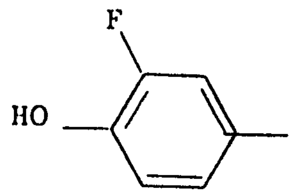 ,

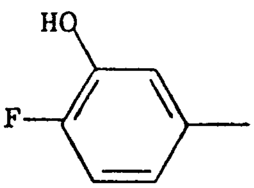 , 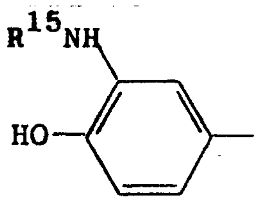

(where $R^{15}$ is HCO-, $CH_3CO-$, $NH_2CO-$, $(CH_3)_2NSO_2-$ or $CH_3SO_2-$),

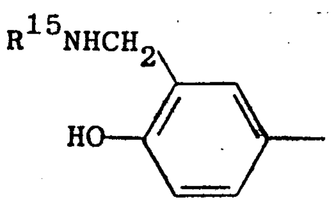

(where $R^{15}$ is as just defined),

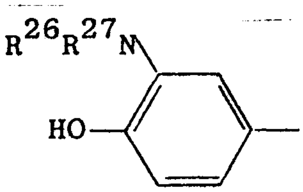

(where $R^{26}$ is hydrogen and $R^{27}$ is methyl),

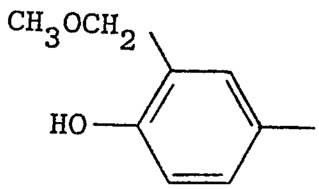

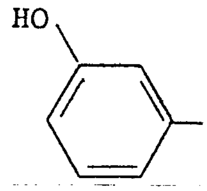 .

or a group of type (f), (g), (h), or (i).

8. A process as claimed in any of claims 1 to 7 for the production of compounds in which $R^1$ and $R^2$ each represent a hydrogen atom, $R^{20}$ is a methyl or ethyl group, Ar is a phenyl group, X is the group $-(CH_2)_5-$, Y is the group $-(CH_2)_2-$ or $-(CH_2)_3)-$ and Q is the group

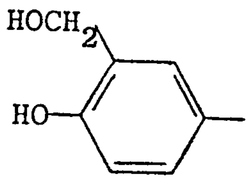

or